# EUROPEAN PATENT APPLICATION

(11) **EP 4 144 842 A1**
(43) Date of publication of application: **08.03.2023**
(21) Application number: 20933801.1
(22) Date of filing: 29.05.2020
(51) Int. Cl.: C12N 11/089, C12N 11/098, C12N 11/18

(54) **PEI IMMOBILIZED ENZYME, AND PREPARATION METHOD THEREFOR AND USE THEREOF**

(30) Priority: 29.04.2020 CN 202010353558
(71) Applicant: Asymchem Life Science (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); RAJASEKAR, Vyasarayani Williams, Morrisville North Carolina 27560 (US); CUI, Yuxia, Tianjin 300457 (CN); ZHAO, Jiadong, Tianjin 300457 (CN); GAO, Yanyan, Tianjin 300457 (CN); FU, Han, Tianjin 300457 (CN)
(74) Representative: V.O.
(86) International application number: PCT/CN2020/093529
(87) International publication number: WO 2021/217774

(57) **Abstract**

Provided are an immobilized enzyme, and a preparation method and a use thereof. The immobilized enzyme includes activated PEI and an enzyme covalently bonded to the activated PEI, herein the enzyme is selected from any one of a transaminase, a ketoreductase, a monooxygenase, an ammonia lyase, an ene-reductase, an imine reductase, an amino acid dehydrogenase and a nitrilase.

## Description

### Technical Field

The present invention relates to the field of immobilized enzymes, in particular to an immobilized enzyme, a preparation method and a use thereof.

### Background

Applications of microbial cells or isolated enzymes or engineered enzymes lead to significant progress of biocatalysis, and changes of manufacturing modes. Many types of enzymes such as an acyltransferase, an amidase, a transaminase, a ketoreductase, an oxidase, a monooxygenase and a hydrolase are used in reactions involved for production of antibiotics, herbicides, pharmaceutical intermediates and new age therapeutic agents.

While a free enzyme is used as a biocatalyst, there is a great waste of the enzymes, because it is very difficult to recover a water-soluble enzyme. However, a water-insoluble immobilized enzyme may be easily recovered by very simple filtration after each cycle.

It is well-known to use a carrier or a support to immobilize the free enzyme. However, a solid-phase carrier has the disadvantages of high cost and limited quantity transfer, and the preparation of the carrier or the support, especially a polymer resin, may cause the serious environmental pollution. Most of methods for immobilizing the enzymes on solid carriers have the inherent disadvantage: low enzyme load and low enzyme specificity. One method to overcome this disadvantage is to purify the enzyme to avoid protein pollution or enzyme load, but the purification cost of the enzyme is also high, and the cost of the immobilized biocatalyst is further increased.

Carrier-free immobilization methods for enzymes such as a lipase, a penicillin acylase, a protease, and an aminoacylase are already reported at present. However, without the carrier support, an immobilized enzyme without containing a carrier may not resist a shearing force and a stirring condition due to the lower mechanical stability, and also cause a filtration problem, so it may not be fully satisfied with mass production.

Moreover, the immobilized enzyme method without containing the carrier is not suitable for some sensitive enzymes, such as a transaminase (TA), a ketoreductase (KRED), a cyclohexanone monooxygenase (CHMO), a phenylalanine ammonia lyase (PAL), an ene-reductase (ERED), an imine reductase (IRED), an amino acid dehydrogenase (AADH) and a nitrilase (Nitrilase).

Therefore, it is necessary to develop a carrier-free immobilized enzyme method, as to improve sensitive enzyme activity and its recycling utilization rate.

### Summary

A main purpose of the present invention is to provide an immobilized enzyme, a preparation method and a use thereof, as to solve a problem in the prior art that it is difficult to achieve the activity of a sensitive enzyme and its cyclic utilization.

In order to achieve the above purpose, according to one aspect of the present invention, an immobilized enzyme is provided, and the immobilized enzyme includes an activated cationic polymer and an enzyme covalently bound to the activated cationic polymer, the activated cationic polymer is a polyetherimide (PEI) activated by a cofactor or a cross-linking agent, and the cross-linking agent is a polyol-treated or untreated cross-linking agent; the enzyme is selected from any one of the followings: a transaminase, a ketoreductase, a monooxygenase, an ammonia lyase, an ene-reductase, an imine reductase, an amino acid dehydrogenase and a nitrilase.

Further, the transaminase is derived from *B.thuringiensis, Arthrobacter citreus* or *Chromobacterium violaceum DSM30191;* preferably, the ketoreductase is derived from *Acetobacter sp. CCTCC M209061* or *Candida macedoniensis.* AKU4588; preferably, the monooxygenase is a cyclohexanone monooxygenase derived from *Brachymonas petroleovorans* or *Rhodococcus ruber-SD1;* preferably, the ammonia lyase is a phenylalanine ammonia lyase derived from *photorhabdus luminescens* or *Solenostemon scutellarioides;* preferably, the ene-reductase is derived from *Saccharomyces cerevisiae* or *Chryseobacterium sp.CA49;* preferably, the imine reductase is derived from *Streptomyces sp.* or *Bacillus cereus;* preferably, the amino acid dehydrogenase is a leucine dehydrogenase derived from *Bacillus cereus* or a phenylalanine dehydrogenase derived from *Bacillus sphaericus;* and preferably, the nitrilase is derived from *Aspergillus niger CBS 513.88* or *Neurospora crassa OR74A.*

Further, the molecular weight of PEI is 3 KDa~600 KDa; preferably, the mass ratio of the enzyme to PEI in the immobilized enzyme is 0.1~1.5:1, and more preferably, the mass ratio is 0.2~1:1.

Further, the cofactor is a pyridoxal phosphate (PLP), a nicotinamide adenine dinucleotide (NAD) or a nicotinamide adenine dinucleotide phosphate (NADP), and the cross-linking agent is a glutaraldehyde; and preferably, the polyol is a polyethylene glycol (PEG), and more preferably, PEG is PEG400~6000.

Further, the immobilized enzyme further includes a cofactor of the enzyme, the cofactor is covalently bound to PEI, and preferably the cofactor is PLP, NAD or NADP.

Further, there is one or more enzymes, there is one or more cofactors, the one or more enzymes correspond to the one or more enzymes, and preferably, the mass ratio of the cofactor to PEI in the immobilized enzyme is 1~80:120, more preferably 1~60:100.

According to a second aspect of the present application, a preparation method for an immobilized enzyme is provided, and the preparation method includes: activating a cationic polymer with an activator, to obtain an activated cationic polymer; and immobilizing an enzyme with the activated cationic polymer, to obtain the immobilized enzyme, herein, the activator is a polyol-unmodified cross-linking agent, a polyol-modified cross-linking agent or a cofactor; and the enzyme is selected from any one of the followings: a transaminase, a ketoreductase, a monooxygenase, an ammonia lyase, an ene-reductase, an imine reductase, an amino acid dehydrogenase and a nitrilase.

Further, before activating PEI with the activator, the preparation method further includes a step of pre-treating PEI, and preferably, the pre-treating includes: diluting PEI with pure water until the final concentration is 1 g/100 mL~8 g/100 mL, and more preferably 1 g/100 mL~5 g/100 mL, to obtain diluted PEI; and adjusting pH of the diluted PEI to 5-11, and more preferably 7.0-10.0, to obtain pre-treated PEI.

Further, the enzyme is a precipitating enzyme, the cross-linking agent is a glutaraldehyde, and the activator is glutaraldehyde or PEG-modified glutaraldehyde; preferably, in the pre-treating step, pH of the diluted PEI is adjusted to 5.0-11.0, preferably 7.0-10.0, to obtain the pre-treated PEI; more preferably, the step of activating PEI with the activator to obtain the activated PEI includes: adding the glutaraldehyde or the PEG-modified glutaraldehyde dropwise to the pre-treated PEI, and controlling the final concentration of the glutaraldehyde or the PEG-modified glutaraldehyde in a system to be 0.1 g/100 mL~4 g/100 mL, preferably 0.3 g/100 mL~2 g/100 mL, to obtain activated PEI.

Further, the precipitating enzyme is obtained by precipitating the enzyme with a precipitating agent, and the precipitating agent is selected from an organic solvent, an ammonium sulfate, PEG400-6000 or PEI with a molecular weight of 3 KDa~70 KDa.

Further, in the process of dropwise adding the glutaraldehyde or the PEG-modified glutaraldehyde to the pre-treated PEI, the dropping rate of the glutaraldehyde or the PEG-modified glutaraldehyde is controlled to be 10~50 mL/min, and preferably, in the process of dropwise adding, it further includes a step of stirring, preferably the temperature of stirring is 4~25°C, the speed of stirring is 50~400 rpm, further preferably 100~300 rpm, and the time of stirring is 1~12 h, further preferably 2~10 h.

Further, the step of binding the enzyme with the activated PEI to obtain the immobilized enzyme includes: adding the activated PEI dropwise to the precipitating enzyme, to obtain a mixture; and performing standing, centrifuging, sieving and drying treatments on the mixture sequentially, to obtain the immobilized enzyme; preferably, the mass ratio of the enzyme to the activated PEI is 0.2~1:1; preferably, the standing time is 1~5 hours; preferably, the sieving is sieving with a sieve of 20~50 meshes; preferably, the drying is natural air-drying, more preferably air-drying overnight; preferably, after the mixture is dried and before the PEI immobilized enzyme is obtained, the preparation method further includes: a step of washing the dried mixture with a phosphate buffer, water and the phosphate buffer sequentially, each washing is repeated for 3-5 times, and pH of the phosphate buffer is 7.0∼8.0.

Further, the enzyme is a free enzyme, the activator is a cofactor, and the cofactor is PLP, NAD or NADP; preferably, in the step of pre-treating, purified water is used to adjust the concentration of the diluted PEI to be 1 g/100 mL~8 g/100 mL, preferably 1 g/100 mL~3 g/100 mL, herein pH is 6~11, and more preferably, pH is 8.0~11.0, to obtain the pre-treated PEI; more preferably, the step of using the activator to activate PEI to obtain the activated PEI includes: adding the cofactor to the pre-treated PEI, and controlling the final concentration of the cofactor to be 0.3~20 mg/mL, further preferably the final concentration of the cofactor is 1~10 mg/mL, and then stirring for 10~600 min, more preferably stirring for 30~180 min, to obtain the activated PEI.

Further, the free enzyme is enzyme solution of one enzyme or mixed solution of more enzymes, and the cofactor is a cofactor corresponding to the more enzymes.

Further, the step of immobilizing the enzyme with the activated PEI to obtain the immobilized enzyme includes: at 4~25°C, adding the activated PEI dropwise to enzyme solution of the free enzyme, to obtain a PEI adsorption enzyme; adding a cross-linking agent dropwise to the PEI adsorption enzyme for immobilization, to obtain a PEI immobilized enzyme; preferably, the concentration of the free enzyme is 0.05∼0.3 g/mL; and preferably, the mass ratio of PEI to the free enzyme is 0.2~1:1.

Further, the step of adding the cross-linking agent dropwise to the PEI adsorption enzyme for immobilization to obtain the PEI immobilized enzyme includes: adding the cross-linking agent dropwise to the PEI adsorption enzyme, and controlling the final concentration of the cross-linking agent to be 0.2 g/100 mL~1 g/100 mL, to obtain a substance be immobilized; performing standing, centrifuging and drying on the substance be immobilized sequentially, to obtain a dried enzyme; and washing the dried enzyme, to obtain the immobilized enzyme; preferably, the standing time is 10~300 min, further preferably 30~120 min, the drying is natural air-drying, the washing is washing for 3~5 times with a phosphate buffer, washing for 3~5 times with water and washing for 3~5 times with the phosphate buffer, and the phosphate buffer contains NaCl, herein the concentration of NaCl is 0.5~1 M.

Further, the transaminase is derived from *B.thuringiensis, Arthrobacter citreus* or *Chromobacterium violaceum DSM30191;* preferably, the ketoreductase is derived from *Acetobacter* sp. CCTCC M209061 or *Candida macedoniensis.* AKU4588; preferably, the monooxygenase is a cyclohexanone monooxygenase derived from *Brachymonas petroleovorans* or *Rhodococcus ruber-SD1;* preferably, the ammonia lyase is a phenylalanine ammonia lyase derived from *photorhabdus luminescens* or *Solenostemon scutellarioides;* preferably, the ene-reductase is derived from *Saccharomyces cerevisiae* or *Chryseobacterium sp.CA49;* preferably, the imine reductase is derived from *Streptomyces sp.* or *Bacillus cereus;* preferably, the amino acid dehydrogenase is a leucine dehydrogenase derived from *Bacillus cereus* or a phenylalanine dehydrogenase derived from *Bacillus sphaericus;* and preferably, the nitrilase is derived from *Aspergillus niger CBS 513.88* or *Neurospora crassa OR74A.*

According to a third aspect of the present application, an application of any one of the above immobilized enzymes or the immobilized enzyme prepared by any one of the above preparation methods in a biocatalytic reaction is provided.

Further, the biocatalytic reaction is a batch reaction or a continuous reaction.

A technical scheme of the present invention is applied, and by immobilizing the enzyme with PEI, the enzyme is immobilized between polymer network-like structures formed by PEI, the mechanical stability is improved, the immobilization of the enzyme is achieved, and in the process of immobilization, the enzyme is avoided from directly covalently binding to the cross-linking agent such as the glutaraldehyde so as to reduce the activity.

### Detailed Description of the Embodiments

It should be noted that embodiments in the present application and features of the embodiments may be combined with each other in the case without conflicting. The present invention is described in detail below with reference to the embodiments.

As mentioned in the background, although some immobilized enzymes without carrier support are already reported in the prior arts, it is not suitable for sensitive enzymes. Therefore, it is still necessary to develop a new immobilization mode, as to improve the activity of the sensitive enzymes and its recycling efficiency. In a typical embodiment of the present application, an immobilized enzyme is provided, and the immobilized enzyme includes an activated cationic polymer and an enzyme covalently bound to the activated cationic polymer, the activated cationic polymer is PEI activated by a cofactor or a cross-linking agent, the cross-linking agent is a polyol-treated or untreated cross-linking agent, and the enzyme is selected from any one of the followings: a transaminase, a ketoreductase, a monooxygenase, an ammonia lyase, an ene-reductase, an imine reductase, an amino acid dehydrogenase and a nitrilase.

In the immobilized enzyme of the present application, network binding is formed between the enzymes, and between the enzyme and PEI, the mechanical stability is improved, not only the immobilization of the enzyme is achieved, but also the activity, stability and recycling times of the sensitive enzymes may be improved.

In the above immobilized enzymes, PEI is a polyethylene imine, PEI for short, and the polyethylene imine, also known as a polyethyleneimine, is a water-soluble high-molecular polymer, soluble in water and ethanol, and insoluble in benzene. A commercially available product is usually aqueous solution with a concentration of 20%~50%. PEI is initially reported to be used as a polycationic non-viral vector. Because of its low price, good transfection effect in vitro and in vivo, and its advantages of convenient use, large-scale production and no immunogenicity, it receives a great attention in an aspect of the non-viral vector as a drug. The PEI polymer contains a plurality of amino groups, and may be cross-linked with a glutaraldehyde to generate various polymeric network-like nanostructures. This network-like structure is dispersed with various functional groups such as an aldehyde group and the amino group, and may be combined with an enzyme protein in a plurality of modes such as a covalent interaction, a hydrogen bond action, an ionic interaction, and a hydrophobic interaction, rather than a single glutaraldehyde with only covalent binding, and the covalent bond action may easily destroy the activity of the enzyme.

Therefore, the above immobilized enzyme of the present application achieves the immobilization of the enzyme by immobilizing the enzyme with PEl, and immobilizing the enzyme between the polymeric network-like structures formed by PEI, and avoids the enzyme from directly covalently binding to the cross-linking agent such as the glutaraldehyde so as to reduce the activity in the immobilization process. The specific molecular weight of the above PEI is not particularly limited, as long as the immobilization of the enzyme may be achieved. In a preferred embodiment of the present application, the molecular weight of PEI is 3 KDa~600 KDa. The specific molecular weight of PEI may be reasonably optimally selected according to the different types of the immobilized enzymes. Within the above molecular weight range, the immobilization effect of existing enzymes is relatively better.

In the above immobilized enzymes, the mass ratio of the enzyme to PEI may be reasonably adjusted according to the different types of the specific enzymes. In a preferred embodiment, the mass ratio of the enzyme to PEI in the immobilized enzyme is 0.1~1.5:1, preferably, the mass ratio is 0.2~1:1. In the range of the mass ratio of 0.1~1.5:1, the number of the recycling times of the immobilized enzyme is relatively high, and in the range of the mass ratio of 0.2~1:1, the number of the recycling times of the immobilized enzyme is higher.

The above cofactor is PLP, NAD or NADP, and the cross-linking agent is the glutaraldehyde; and preferably, the polyol is PEG, and more preferably, PEG is PEG400~6000.

The above immobilized enzyme may be immobilized by a single enzyme, or may be immobilized by a plurality of enzymes. While the plurality of the enzymes is immobilized, it may be co-immobilization of a main enzyme and a coenzyme. In a preferred embodiment of the present application, the immobilized enzyme further includes a cofactor of an enzyme, the cofactor is covalently bound to PEI, and preferably the cofactor is PLP, NAD or NADP. The co-immobilized enzyme of the main enzyme and the coenzyme has the effects of stable catalytic activity and high recycling times.

As mentioned above, the enzyme in the immobilized enzyme of the present application may be one or more, and correspondingly, the cofactor may also be one or more, and the one or more cofactors correspond to the one or more enzymes. Preferably, the mass ratio of the cofactor to PEI in the immobilized enzyme is 1~80:120, more preferably 1~60:100.

While two or more than two enzymes have the different cofactors, the immobilized enzyme contains the different cofactors of respective enzymes; and while two or more than two enzymes have the same cofactor, the amount of the cofactor contained in the immobilized enzyme is rationally matched with the amount of various main enzymes. According to the different amounts of the specific cofactors, the corresponding amounts of PEI are also different.

In a second typical embodiment of the present application, a preparation method for an immobilized enzyme is provided, and the preparation method includes: activating a cationic polymer by using an activator, to obtain an activated cationic polymer; and immobilizing an enzyme with the activated cationic polymer, to obtain the immobilized enzyme, herein, the activator is a polyol-unmodified cross-linking agent, a polyol-modified cross-linking agent or a cofactor; and the enzyme is selected from any one of the followings: a transaminase, a ketoreductase, a monooxygenase, an ammonia lyase, an ene-reductase, an imine reductase, an amino acid dehydrogenase and a nitrilase.

In the above preparation method for the immobilized enzyme, the immobilized enzyme may be obtained by firstly using the glutaraldehyde or the cofactor to activate PEI, and then combining with the enzyme. The method is simple and does not easily affect the activity of the enzyme in the immobilization process, so that the immobilized enzyme has the high activity, good stability and many recycling times.

In order to activate PEI more effectively, in a preferred embodiment of the present application, before activating PEI by using the activator, the preparation method further includes a step of pre-treating PEI, and more preferably, the pre-treating includes: diluting PEI with water until the final concentration is 1 g/100 mL~8 g/100 mL, and further preferably 1 g/100 mL~5 g/100 mL, to obtain diluted PEI; and adjusting pH of the diluted PEI to 5-11, and more preferably 7.0~10.0, to obtain pre-treated PEI. A reason for the dilution is: to reduce the viscosity and concentration of PEI, it is beneficial to the activation process and prevents the formation of precipitation due to the excessive concentration. A purpose of pH adjustment is that: too high or too low pH may cause the denaturation and inactivation of the enzyme, and may also affect the degree of binding between each level of the amino functional group on PEI and the glutaraldehyde, and too high pH may also easily cause the formation of precipitation in the activation process.

In the above preparation method, according to whether the enzyme is a precipitating enzyme or a free enzyme, its activators are also different, and the specific preparation steps are also slightly different. In a preferred embodiment, the enzyme is a precipitating enzyme, the cross-linking agent is a glutaraldehyde, and the activator is glutaraldehyde or PEG-modified glutaraldehyde. More preferably, in the pre-treating step, pH of the diluted PEI is adjusted to 5.0~11.0, preferably 7.0~10.0, to obtain the pre-treated PEI. In a more preferred embodiment, the above step of activating PEI with the activator to obtain the activated PEI includes: adding the glutaraldehyde or the PEG-modified glutaraldehyde dropwise to the pre-treated PEI, and controlling the final concentration of the glutaraldehyde or the PEG-modified glutaraldehyde in a system to be 0.1 g/100 mL~4 g/100 mL, preferably 0.3 g/100 mL~2 g/100 mL, to obtain activated PEI.

While the enzyme is a precipitating enzyme, glutaraldehyde or PEGylated glutaraldehyde is used to activate PEI, and by controlling the concentration of the glutaraldehyde or PEGylated glutaraldehyde within the above preferred range, PEI with the better activation performance may be obtained, thereby it is more beneficial to the effective binding with the enzyme.

The step of activating PEI with the glutaraldehyde or PEGylated glutaraldehyde does not have a special requirement, as long as the activity efficiency of the glutaraldehyde or PEGylated glutaraldehyde on PEI is controlled to be the best. In order to activate PEI more fully and uniformly, in a preferred embodiment, in the process of dropwise adding the glutaraldehyde or PEGylated glutaraldehyde to the pre-treated PEI, the dropping rate of the glutaraldehyde or PEGylated glutaraldehyde is controlled to be 10~50 mL/min, more preferably, in the process of dropwise adding, it further includes a step of stirring, preferably the stirring temperature is 4~25 °C, and the stirring speed is 50~400 rpm, further preferably 100-300 rpm, and the stirring time is 1~12 h, further preferably 2~10 h.

The above precipitating enzyme may be formed by an existing method. In the present application, it is preferably obtained by precipitating the enzyme with a precipitating agent. The selection of the precipitating agent includes but not limited to an organic solvent (such as an acetonitrile), an ammonium sulfate, PEG400~6000 or PEI with a molecular weight of 3 KDa~70 KDa. More preferably, PEG400~6000 or PEI with the molecular weight of 3 KDa~70 KDa is used to obtain the precipitating enzyme. It should be noted that while the precipitating agent is PEI of 3 KDa~70 KDa, since the precipitating enzyme contains PEI, and the physical adsorption of PEI and the enzyme is achieved during the precipitation process, it may be directly covalently bound to the enzyme to form the immobilized enzyme under the cross-linking effect of the glutaraldehyde at this time. Certainly, if it reacts with glutaraldehyde-activated PEI at this time, a precipitating enzyme aggregate is also covalently bound to PEI by the arm action of the cross-linking agent, to form network-like binding between the enzymes, and between the enzyme and PEI.

While the precipitating enzyme is formed by the organic solvent, the ammonium sulfate, PEG400~6000 and the like, it needs to be combined with the glutaraldehyde-activated PEI. In a preferred embodiment, the above step of binding the enzyme with the activated PEI to obtain the immobilized enzyme includes: dropwise adding the activated PEI to the precipitating enzyme, and controlling a pH value to be 5-11 in the process of dropwise adding, preferably 7.0~9.0, to obtain a mixture; and performing standing, centrifuging, sieving and drying treatments on the mixture sequentially, to obtain the immobilized enzyme; and more preferably, the mass ratio of the enzyme to the activated PEI is 0.2~1:1.

While the pH value is in the range of 5-11, it has the beneficial effect of improving the firmer binding of PEI and the activator on the basis of guaranteeing the enzymatic activity, and while the pH value is controlled in the range of 7.0~9.0 in the process of dropwise adding, it has the more excellent effects that the activity on the enzyme is best, the degree of binding PEI and the activator is best and the operation is easier. The mass ratio of the enzyme to the activated PEI is in the range of 0.2~1:1, it may achieve the technical effect of the obtained immobilized enzyme with the highest activity on the basis without wasting the enzyme.

In the above preferred embodiment, the effect of standing is to further strengthen the internal interaction between an enzyme molecule and the activated PEI to form the immobilized enzyme with the higher mechanical strength. It is sieved to form the immobilized enzyme with a uniform particle size. In a preferred embodiment, the above standing time is 1-5 hours; more preferably, the sieving is sieving with a sieve of 20-50 meshes; more preferably, the drying is natural air-drying, more preferably air-drying overnight; more preferably, after the mixture is dried and before the PEI immobilized enzyme is obtained, the above preparation method further includes: a step of washing the dried mixture with a phosphate buffer, water and the phosphate buffer sequentially, each washing is repeated for 3-5 times, pH of the phosphate buffer is 7.0∼8.0, and the phosphate buffer includes NaCl, herein the concentration of NaCl 0.5-1 M.

In the above preferred embodiment, the washing of the mixture using the phosphate buffer may prevent washing liquid from adversely affecting the activity of the enzyme. The buffer within the above concentrations and pH ranges does not affect the enzyme activity.

While the enzyme is a free enzyme, in a preferred embodiment, the enzyme is a free enzyme, the activator is a cofactor, and the cofactor is PLP, NAD or NADP. Under the action of the above cofactor, PEI is activated, and the activated PEI may effectively adsorb the free enzyme, and then the cross-linking immobilization of PEI and the enzyme may be achieved under the action of the cross-linking agent.

In a preferred embodiment, in the above pre-treatment step, purified water is used to adjust the concentration of diluted PEI to be 1 g/100mL~8 g/100mL, preferably 1 g/100 mL~3 g/100 mL, pH is 6-11, preferably pH is 8.0~11.0, and the pre-treated PEI is obtained. For the free enzyme, the concentration and pH of the diluted PEI are within the above ranges, so that the effective functional group distribution of the activated PEI is more beneficial to the binding with the enzyme molecule, the immobilized enzyme has a good precipitation state, and it is more beneficial to separation from the solution.

For the immobilization of the free enzyme, in a preferred embodiment, the step of using the activator to activate the PEI to obtain the activated PEI includes: adding the cofactor to the pre-treated PEI, and controlling the final concentration of the cofactor to be 0.3~20 mg/mL, further preferably the final concentration of the cofactor is 1~10 mg/mL, and then stirring for 10~600 min, more preferably stirring for 30-180 min, to obtain the activated PEI.

The final concentration of the cofactor is controlled within the above range, it has the effect that the degree of activating PEI is best on the basis without wasting the cofactor and it is more beneficial to the binding of the enzyme molecule in a later stage. The added cofactor is stirred, and it is beneficial to fully achieve the activation of PEI by the cofactor.

The above free enzyme is enzyme solution of an enzyme or mixed solution of more enzymes, and correspondingly, the cofactor is a cofactor corresponding to one enzyme or more enzymes.

In a preferred embodiment, the step of immobilizing the enzyme with the activated PEI to obtain the immobilized enzyme includes: at 4~25°C, adding the activated PEI dropwise to enzyme solution of the free enzyme, to obtain a PEI adsorption enzyme; adding a cross-linking agent dropwise to the PEI adsorption enzyme for immobilization, to obtain a PEI immobilized enzyme; more preferably, the concentration of the free enzyme is 0.05~0.3 g/mL; and more preferably, the mass ratio of PEI to the free enzyme is 0.2~1:1.

The concentration of the free enzyme is controlled in the range of 0.05~0.3 g/mL, it is beneficial to the immobilized enzyme with the suitable particle size and uniform distribution, and the immobilized enzyme is beneficial to be separated from the solution. The mass ratio of the activated PEI to the free enzyme is controlled in the range of 0.2~1:1, and it has the effect of forming the immobilized enzyme with the most profitable recovery on the basis without wasting the free enzyme.

In a preferred embodiment, the step of adding the cross-linking agent dropwise to the PEI adsorption enzyme for immobilization to obtain the PEI immobilized enzyme includes: adding the cross-linking agent dropwise to the PEI adsorption enzyme, and controlling the final concentration of the cross-linking agent to be 0.2 g/100 mL~1 g/100 mL, to obtain a substance be immobilized; performing standing, centrifuging and drying on the substance be immobilized sequentially, to obtain a dried enzyme; and washing the dried enzyme, to obtain the immobilized enzyme; preferably, the standing time is 10-300 min, further preferably 30-120 min, the drying is natural air-drying, the washing is washing for 3-5 times with a phosphate buffer, washing for 3-5 times with water and washing for 3-5 times with the phosphate buffer, and the phosphate buffer contains NaCl, herein the concentration of NaCl is 0.5-1 M.

The final concentration of the cross-linking agent is controlled in the range of 0.2 g/100 mL~1 g/100 mL, and it has the effect of guaranteeing the formation of the firmly bound immobilized enzyme and the highest recovery of the enzyme activity. The functions of standing and washing and the like here are the same as those described above.

In a third typical embodiment of the present application, an application of any one of the above immobilized enzymes or the immobilized enzyme prepared by any one of the above preparation methods in a biocatalytic reaction is provided. The immobilized enzyme has the advantages of high stability and high recycling efficiency, so it may be repeatedly used for many times in the biocatalytic reaction.

In a more preferred embodiment, the biocatalytic reaction used by the above immobilized enzyme is a batch reaction or a continuous reaction, more preferably the continuous reaction. The immobilized enzyme has the high recycling efficiency, so it is suitable for the continuous biocatalytic reaction, to improve the reaction efficiency.

The beneficial effects of the present application are further described below with reference to specific embodiments.

**Table 1:**

| Abbreviation | Enzyme | Source (gene ID number) |
|---|---|---|
| TA-Bt | Transaminase | *B.thuringiensis* (WP_000648390) |
| TA-Ac | Transaminase | *Arthrobacter citreus* (PDB:5G2P_A) |
| TA-Cv | Transaminase | *Chromobacterium violaceum DSM30191* (WP_011135573.1) |
| KRED-Ac | Ketoreductase | *Acetobacter sp.* CCTCC M209061 (WP_020944327.1) |
| KRED-Cm | Ketoreductase | Candida macedoniensis. AKU4588 (BAE46987.1) |
| CHMO-Bp | Cyclohexanone monooxygenase | *Brachymonas petroleovorans* (AAR99068.1) |
| CHMO-Rr | Cyclohexanone monooxygenase | *Rhodococcus ruber-SD1* (AAL14233.1) |
| CHMO-Rs | Cyclohexanone monooxygenase | *Rhodococcus sp. Phil* (AAN37 4941) |
| ERED-Sc | Ene Reductase | *Saccharomyces cerevisiae* (AJU28279.1) |
| ERED-Chr | Ene Reductase | *Chryseobacterium sp.* CA49 (ALE60336.1) |
| NIT-An | Nitrilase | *Aspergillus niger CBS 513.88* (XP_001397369.1) |
| NIT-Nc | Nitrilase | *Neurospora crassa OR74A* (XP_011393515.1) |
| IRED-Str | Imine Reductase | *Streptomyces sp.* (WP_086772432) |
| IRED-Bc | Imine Reductase | *Bacillus cereus* (WP_095755701.1) |
| PAL-An | Phenylalanine Ammonia lyase | *photorhabdus luminescens* (WP_036806208.1) |
| PAL-Ss | Phenylalanine Ammonia lyase | *Solenostemon scutellarioides* (WP_036806208.1) |
| AADH-Bc | Leucine Dehydrogenase | *Bacillus cereus* (WP_000171355.1) |
| AADH-Bs | Phenylalanine Dehydrogenase | *Bacillus sphaericus* (P23307.1) |

**Table 2: Sequences of female parents of some enzymes and mutants thereof**

| TA-Cv | Sequence ID | Sequence |
|---|---|---|
| Female parent | SEQ ID NO: 1 | MQKQRTTSQWRELDAAHHLHPFTDTASLNQAGARVMTRGEGVYL WDSEGNKIIDGMAGLWCVNVGYGRKDFAEAARRQMEELPFYNTFF KTTHPAVVELSSLLAEVTPAGFDRVFYTNSGSESVDTMIRMVRRYW DVQGKPEKKTLIGRWNGYHGSTIGGASLGGMKYMHEQGDLPIPGM AHIEQPWWYKHGKDMTPDEFGVVAARWLEEKILEIGADKVAAFVGE PIQGAGGVIVPPATYWPEIERICRKYDVLLVADEVICGFGRTGEWFG HQHFGFQPDLFTAAKGLSSGYLPIGAVFVGKRVAEGLIAGGDFNHG FTYSGHPVCAAVAHANVAALRDEGIVQRVKDDIGPYMQKRWRETFS RFEHVDDVRGVGMVQAFTLVKNKAKRELFPDFGEIGTLCRDIFFRN |
| | | NLIMRACGDHIVSAPPLVMTRAEVDEMLAVAERCLEEFEQTLKARGL A |
| Mutant 1 (TA-Cv-V1) | SEQ ID NO: 2 | R416T+T7C+S47C+Q380L |
| Mutant 2 (TA-Cv-V2) | SEQ ID NO: 3 | R416T+T7C+S47C+R405E+K90G+A95P+K304D+Q380L+I297L |

**Table 3:**

| TA-Ac | Sequence ID | Sequence |
|---|---|---|
| Female parent | SEQ ID NO: 4 | MGLTVQKINWEQVKEWDRKYLMRTFSTQNEYQPVPIESTEGDYLITP GGTRLLDFFNQLCCVNLGQKNQKVNAAIKEALDRYGFVWDTYATDY KAKAAKIIIEDILGDEDWPGKVRFVSTGSEAVETALNIARLYTNRPLVV TREHDYHGWTGGAATVTRLRSFRSGLVGENSESFSAQIPGSSCSSA VLMAPSSNTFQDSNGNYLKDENGELLSVKYTRRMIENYGPEQVAAVI TEVSQGVGSTMPPYEYVPQIRKMTKELGVLWISDEVLTGFGRTGKW FGYQHYGVQPDIITMGKGLSSSSLPAGAVVVSKEIAAFMDKHRWESV STYAGHPVAMAAVCANLEVMMEENLVEQAKNSGEYIRSKLELLQEKH KSIGNFDGYGLLWIVDIVNAKTKTPYVKLDRNFRHGMNPNQIPTQIIM EKALEKGVLIGGAMPNTMRIGASLNVSRGDIDKAMDALDYALDYLES GEWQQS |
| Mutant1 (TA-Ac-V1) | SEQ ID NO: 5 | L3S+V5S+C60Y+F164L+A178L+S187A+I180V+L370A+G411D+S186 G+Y384F+I389F+V252I+L404Q+E171D |
| Mutant2 (TA-Ac-V2) | SEQ ID NO: 6 | L3S+V5S+C60Y+F164L+A178L+S187A+I180V+L370A+G411D+S186 G+Y384F+I389F+V252I+E424Q+M423K |

**Table 4:**

| KRED-Ac | Sequence ID | Sequence |
|---|---|---|
| Female parent | SEQ ID NO: 7 | MARVAGKVAIVSGAANGIGKATAQLLAKEGAKVVIGDLKEEDGQKAVA EIKAAGGEAAFVKLNVTDEAAWKAAIGQTLKLYGRLDIAVNNAGINYS GSVESTSLEDWRRVQSINLDGVFLGTQVAIEAMKKSGGGSIVNLSSIS GLIGDPMLAAYVASKGGVRLFTKSAALHCAKSGYKIRVNSVHPGYIWT PMVAGLTKEDAAARQKLVDLHPIGHLGEPNDIAYGILYLASDESKFVTG SELVIDGGYTAQ |
| Mutant1 (KRED-Ac-V1) | SEQ ID NO: 8 | E144S+A94N+N156V |
| Mutant2 (KRED-Ac-V2) | SEQ ID NO: 9 | E144S+A94T+N156T |

**Table 5:**

| CHMO-Rs | Sequence ID | Sequence |
|---|---|---|
| Female parent | SEQ ID NO: 10 | MTAQISPTVVDAVVIGAGFGGIYAVHKLHNEQGLTVVGFDK ADGPGGTWYWNRYPGALSDTESHLYRFSFDRDLLQDGTWKTTYI TQPEILEYLESVVDRFDLRRHFRFGTEVTSAIYLEDENLWEVSTDK GEVYRAKYVVNAVGLLSAINFPDLPGLDTFEGETIHTAAWPEGKNL AGKRVGVIGTGSTGQQVITALAPEVEHLTVFVRTPQYSVPVGNRP VTKEQIDAIKADYDGIWDSVKKSAVAFGFEESTLPAMSVSEEERNR IFQEAWDHGGGFRFMFGTFGDIATDEAANEAAASFIRSKIAEIIEDP ETARKLMPTGLYAKRPLCDNGYYEVYNRPNVEAVAIKENPIREVTA KGVVTEDGVLHELDVLVFATGFDAVDGNYRRIEIRGRNGLHINDHW DGQPTSYLGVTTANFPNWFMVLGPNGPFTNLPPSIETQVEWISDT VAYAERNEIRAIEPTPEAEEEWTQTCTDIANATLFTRGDSWIFGANV PGKKPSVLFYLGGLGNYRNVLAGVVADSYRGFELKSAVPVTA |
| Mutant1 (CHMO-Rs-V1) | SEQ ID NO: 11 | F508Y+F435N+L438A+T436S+F280V+S441V |
| Mutant2 (CHMO-Rs-V2) | SEQ ID NO: 12 | F508Y+F435N+L438A+T436S+F280V+S441V+L510V |

**Table 6:**

| CHMO-Rr | Sequence ID | Sequence |
|---|---|---|
| Female parent | SEQ ID NO: 13 | MTTSIDREALRRKYAEERDKRIRPDGNDQYIRLDHVDGWSHDPYM PITPREPKLDHVTFAFIGGGFSGLVTAARLRESGVESVRIIDKAGDF GGVWYWNRYPGAMCDTAAMVYMPLLEETGYMPTEKYAHGPEILE HCQRIGKHYDLYDDALFHTEVTDLVWQEHDQRWRISTNRGDHFTA QFVGMGTGPLHVAQLPGIPGIESFRGKSFHTSRWDYDYTGGDALG APMDKLADKRVAVIGTGATAVQCVPELAKYCRELYVVQRTPSAVDE RGNHPIDEKWFAQIATPGWQKRWLDSFTAIWDGVLTDPSELAIEHE DLVQDGWTALGQRMRAAVGSVPIEQYSPENVQRALEEADDEQME RIRARVDEIVTDPATAAQLKAWFRQMCKRPCFHDDYLPAFNRPNTH LVDTGGKGVERITENGVVVAGVEYEVDCIVYASGFEFLGTGYTDRA GFDPTGRDGVKLSEHWAQGTRTLHGMHTYGFPNLFVLQLMQGAA LGSNIPHNFVEAARVVAAIVDHVLSTGTSSVETTKEAEQAWVQLLL DHGRPLGNPECTPGYYNNEGKPAELKDRLNVGYPAGSAAFFRMM DHWLAAGSFDGLTFR |
| Mutant1 (CHMO-Rr-V1) | SEQ ID NO: 14 | P190L + Y559M + C249V + C393V + C257A + M45T |
| Mutant2 (CHMO-Rr-V2) | SEQ ID NO: 15 | Y559M + P190L + P504V |

The chemical process involved in the reaction of the above enzyme is briefly described as follows:

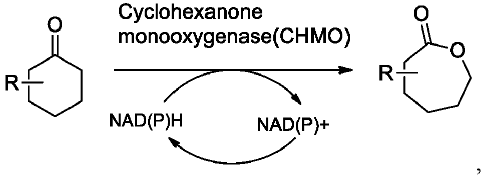

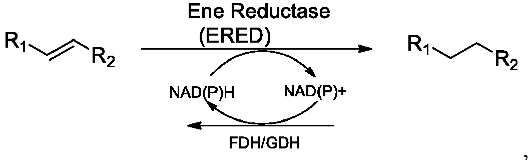

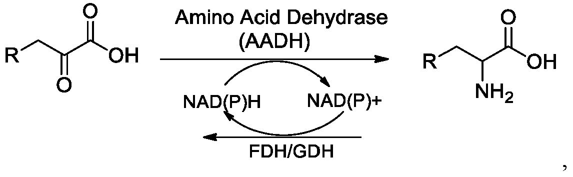

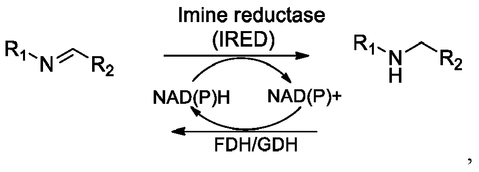

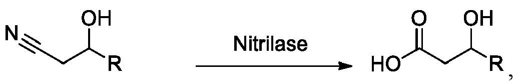

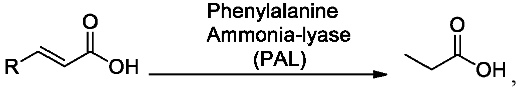

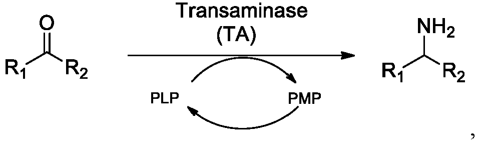

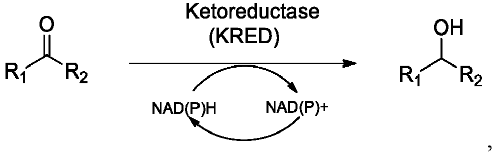

R, R1 and R2 in the above reaction formula may be each independently selected from H, a substituted or unsubstituted alkyl, a substituted or unsubstituted cycloalkyl, a substituted or unsubstituted aralkyl, a substituted or unsubstituted heterocyclyl, a substituted or unsubstituted heterocycloalkyl, or R1 and a heterocycle to which it is linked form a fused ring system.

PB in the following embodiments means the phosphate buffer.

### Embodiment 1: Preparation of glutaraldehyde-activated PEI covalently immobilized enzyme aggregate (GA: Glutaraldehyde, PEI: Polyethylene imine, and GP: GA-activated PEI)

PEG-modified glutaraldehyde: the glutaraldehyde is diluted with water until the final concentration is 20 g/100 mL, and 2-10 g/100 mLof PEG 2000 or PEG 6000 is added, and mixed at a room temperature for 1-3 h for future use.

Preparation of activated PEI solution 1: 2 g of the glutaraldehyde and 4 g of PEI (3-70 KDa) are added to 100 mL of aqueous solution at pH 8.0 and stirred for 3 hours.

Preparation of activated PEI solution 2: 4 g of PEI (3 KDa) is added to 100 mL of aqueous solution at pH 8.0, and PEG-modified glutaraldehyde (the final concentration of the glutaraldehyde in solution is 2 g/100 mL) is stirred for 3 hours.

Immobilization: 10 mL of an enzyme (the enzyme protein content is 100-200 mg/mL, correspondingly containing 3-10 mg/mL of a cofactor) is placed in a round-bottom shake flask, and after being cooled to 5~10°C, it is mixed for 10 min. A precipitating agent such as an ammonium sulfate (45%-65%), ethanol (80%-90%) or PEI (20%-60%) is slowly added, and mixed for 30-60 min, to obtain a precipitating enzyme. 15 mL of activated PEI solution is added dropwise to the precipitating enzyme, and mixed for 10-30 min, and after standing for 1-2 hours, it is centrifuged. An insoluble precipitate is collected, and sieved by a 30-mesh sieve. After being air-dried in a natural environment, it is soaked with 0.1 M PB (pH 7.5, containing 0.5 M NaCl) for 3 hours, and then washed with 0.1 M PB (pH 7.5).

### Embodiment 2: Preparation of cofactor-activated PEI covalently immobilized enzyme aggregate

Preparation of cofactor-activated PEI solution: PEI (Mw=3-70 KDa) is diluted with water until the final concentration of PEI is 1 g/100 mL~2 g/100 mL, and a pH value is 7.0~11.0. According to the needs of the enzyme, 3-10 mg/mL of each enzyme cofactor is added to the PEI solution, and mixed for 30-60 min at a room temperature.

Immobilization: 7.5 mL of cofactor-activated PEI solution is added dropwise to 5 mL of enzyme solution (the enzyme protein content is 30-80 mg/mL, correspondingly also containing the cofactor) at 4-10°C, and after being mixed for 30-60 min, the cross-linking agent glutaraldehyde or PEG-modified glutaraldehyde are added, so that the final concentration of the glutaraldehyde is 0.2-1 g/100 mL. After being mixed for 30-60 min, an insoluble precipitate is collected by centrifugation, and sieved by a 30-mesh sieve. After being naturally air-dried, the precipitate is soaked with 0.1M PB (pH 7.0-8.0, containing 0.5-0.9 M NaCl) for 3 hours, and then washed with 0.1 M PB (pH 7.0-8.0).

### Embodiment 3: Aqueous phase reaction activity verfication of transaminase-glutaraldehyde-activated PEI covalently immobilized enzyme aggregate

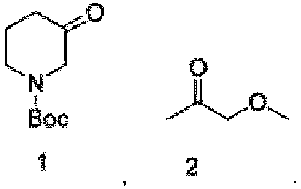

In a 10 mL reaction flask, 0.3 mL of a dimethylsulfoxide (DMSO) is added, 0.1 g of a main raw material 1 or 2 is dissolved, 4 eq of an isopropylamine hydrochloride and 1.0 mg of pyridoxal 5'-phosphate (PLP) are added, and 0.1 M PB 7.0 is added until the final volume of reaction solution is 1 mL, and 0.1 g of an immobilized transaminase (wet, with a water content of 70%-80%) is stirred at 30°C for 16-20 hours. The conversion rate of a system is detected by a high performance liquid chromatography (HPLC), and reaction data is as follows.

**Table 7:**

| Enzyme | Precipitating agent | Cross-linking agent | Conversion rate (%) | Recycling times (times) |
|---|---|---|---|---|
| TA-Ac | Ammonium sulfate | GA | >92% | 7 |
| | | GA-activated PEI | >97% | 10 |
| | | PEG-GA activated PEI | >97% | 12 |
| | Acetonitrile | GA-activated PEI | >97% | 12 |
| | | PEG-GA activated PEI | >97% | 16 |
| | PEG 6000 | GA-activated PEI | >97% | 13 |
| | | PEG-GA activated PEI | >97% | 16 |
| TA-Ac-V1 | PEG 6000 | GA-activated PEI | >97% | 18 |
| | | PEG-GA activated PEI | >97% | 21 |
| TA-Ac-V2 | PEG 6000 | GA-activated PEI | >97% | 23 |
| | | GA-activated PEI | >97% | 25 |
| TA-Cv | Acetonitrile | GA-activated PEI | >97% | 6 |
| | | PEG-GA activated PEI | >97% | 10 |
| | Ammonium sulfate | GA | >97% | 8 |
| | | GA-activated PEI | >97% | 15 |
| | | PEG-GA activated PEI | >97% | 17 |
| | Acetonitrile | GA-activated PEI | >97% | 18 |
| | | PEG-GA activated PEI | >97% | 19 |
| | PEG 6000 | GA-activated PEI | >97% | 17 |
| | | PEG-GA activated PEI | >97% | 19 |
| TA-Cv-V1 | PEG 6000 | GA-activated PEI | >97% | 22 |
| | | PEG-GA activated PEI | >97% | 23 |
| TA-Cv-V2 | PEG 6000 | GA-activated PEI | >97% | 25 |
| | | PEG-GA activated PEI | >97% | 27 |

### Embodiment 4: Investigation on concentration of enzyme solution, PEI concentration and GA concentration in transaminase-glutaraldehyde-activated PEI covalently immobilized enzyme method

PEG6000 is used as a precipitating agent, and the rest of the preparation process is the same as that of Embodiment 1, and the different PEI concentration, cross-linking agent concentration, and ratio of the enzyme to PEI are set, to prepare the corresponding immobilized enzyme.

In a 10 mL reaction flask, 0.3 mL of DMSO is added, 0.1 g of a main raw material 1 or 2 is added, 4 eq of an isopropylamine hydrochloride and 1.0 mg of PLP are added, and 0.1 M PB 7.0 is added until the final volume of reaction solution is 1 mL, and then 0.1 g of an immobilized transaminase (wet, with a water content of 70%- 80%) is added, and stirred at 30°C for 16-20 hours. The conversion rate of a system is detected by HPLC, and results are shown in the following table.

**Table 8:**

| Enzyme | PEI concentration, g/100mL | Ratio of enzyme to PEI | Cross-linking agent | Cross-linking agent concentration, g/100mL | Conversion rate (%) | Recycling times (times) |
|---|---|---|---|---|---|---|
| TA-Cv-V2 | 0.5 | 0.5: 1 | GA-activated PEI | 1 | >97% | 20 |
| | 1 | 0.5: 1 | GA-activated PEI | 1 | >97% | 25 |
| | 3 | 0.2: 1 | GA-activated PEI | 1 | >97% | 25 |
| | 3 | 0.5: 1 | GA-activated PEI | 1 | >97% | 26 |
| | 3 | 1: 1 | GA-activated PEI | 1 | >97% | 25 |
| | 3 | 1.5: 1 | GA-activated PEI | 1 | >97% | 19 |
| | 3 | 1.7: 1 | GA-activated PEI | 1 | >97% | 18 |
| | 5 | 0.5: 1 | GA-activated PEI | 1 | >97% | 25 |
| | 7 | 0.5: 1 | GA-activated PEI | 1 | >97% | 25 |
| | 8 | 0 5: 1 | GA-activated PEI | 1 | >97% | 21 |
| | 3 | 0.5: 1 | GA-activated PEI | 0.1 | >97% | 17 |
| | 3 | 0.5: 1 | GA-activated PEI | 0.3 | >97% | 23 |
| | 3 | 0.5: 1 | GA-activated PEI | 1 | >97% | 26 |
| | 3 | 0.5: 1 | GA-activated PEI | 1.5 | >97% | 26 |
| | 3 | 0.5: 1 | GA-activated PEI | 2 | >97% | 21 |
| | 3 | 0.5: 1 | GA-activated PEI | 4 | >97% | 13 |
| | 3 | 0.5: 1 | PEG-GA activated PEI | 0.3 | >97% | 15 |
| | 3 | 0.5: 1 | PEG-GA activated PEI | 1 | >97% | 26 |
| | 3 | 0.5: 1 | PEG-GA activated PEI | 1.5 | >97% | 24 |
| | 3 | 0.5: 1 | PEG-GA activated PEI | 2 | >97% | 22 |
| | 3 | 0.5: 1 | PEG-GA activated PEI | 4 | >97% | 12 |

The results in the above table show that the PEI concentration of 1-7 g/100 mL is suitable; the best ratio of the enzyme to PEI is 0.2-1:1; and the stability is best while the cross-linking agent concentration is 0.5~1.5 g/100 mL.

### Embodiment 5: Aqueous phase reaction activity verification of transaminase-cofactor-activated PEI covalently immobilized enzyme aggregate

Activity verification is performed by using the same aqueous phase reaction as in Embodiment 3, and results are shown in the following table. The results show that the PEI concentration of 1%-5% is more suitable; the cofactor concentration is >5 mg/mL, there is basically no difference, and for the sake of saving, 5-10 mg/mL is selected; and the cross-linking agent concentration of 0.5-1% is the most suitable. The activity of the immobilized enzyme prepared by the PEG-modified cross-linking agent is slightly improved.

**Table 9: Aqueous reaction overview of transaminase-cofactor-activated PEI covalently immobilized enzyme**

| Enzyme | PEI concentration, g/100mL | Cofactor PLP concentration, mg/mL | Cross-linking agent | Cross-linking agent concentration, g/100mL | Conversion rate (%) | Recycling times (times) |
|---|---|---|---|---|---|---|
| TA-Ac-V2 | 1 | 10 | GA | 1 | >97% | 15 |
| | 3 | 10 | GA | 1 | >97% | 17 |
| | 5 | 10 | GA | 1 | >97% | 16 |
| | 8 | 10 | GA | 1 | >97% | 11 |
| | 2 | 10 | GA | 0.2 | >97% | 17 |
| | 2 | 10 | GA | 0.5 | >97% | 19 |
| | 2 | 10 | GA | 1 | >97% | 18 |
| | 2 | 10 | GA | 2 | >97% | 13 |
| | 2 | 0 | GA | 1 | >97% | 3 |
| | 2 | 0.1 | GA | 1 | >97% | 10 |
| | 2 | 0.3 | GA | 1 | >97% | 16 |
| | 2 | 1 | GA | 1 | >97% | 16 |
| | 2 | 5 | GA | 1 | >97% | 18 |
| | 2 | 10 | GA | 1 | >97% | 18 |
| | 2 | 20 | GA | 1 | >97% | 18 |
| | 2 | 30 | GA | 1 | >97% | 18 |
| TA-Cv-V2 | 2 | 10 | GA | 1 | >97% | 23 |
| | 2 | 10 | PEG-GA | 0.2 | >97% | 24 |
| | 2 | 10 | PEG-GA | 0.5 | >97% | 25 |
| | 2 | 10 | PEG-GA | 1 | >97% | 25 |
| | 2 | 10 | PEG-GA | 2 | >97% | 24 |

### Embodiment 6: aqueous phase reaction activity verification of ketoreductase-glutaraldehyde-activated PEI covalently immobilized enzyme aggregate

A ketoreductase-glutaraldehyde-activated PEI covalently immobilized enzyme is prepared according to the method of Embodiment 1.

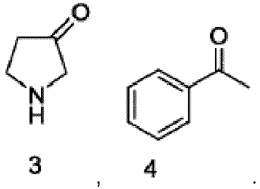

In a 10 mL reaction flask, 0.5 mL of isopropanol (IPA) is added, 0.1 g of a main material 3 or 4 is dissolved, 0.5 mL of 0.1 M PB 7.0 and 1-10 mg of a nicotinamide adenine dinucleotide (NAD+) are added, and then 0.1 g of the immobilized enzyme (wet, with a water content of 70-80%) is added, and it is stirred at 30°C for 16-20 hours. The conversion rate of a system is detected by a gas chromatography (GC), and reaction data is as follows.

**Table 10: aqueous reaction overview of ketoreductase-glutaraldehyde-activated PEI covalently immobilized enzyme**

| Enzyme | Precipitating agent | Cross-linking agent | Conversion rate (%) | Recycling times |
|---|---|---|---|---|
| KRED-Ac | Ammonium sulfate | GA | >99% | 4 |
| | | GA-activated PEI | >99% | 7 |
| | | PEG-GA activated PEI | >99% | 9 |
| | Acetonitrile | GA-activated PEI | >99% | 10 |
| | | PEG-GA activated PEI | >99% | 12 |
| | PEG 6000 | GA-activated PEI | >99% | 8 |
| | | PEG-GA activated PEI | >99% | 11 |
| KRED-Ac-V1 | PEG 6000 | GA | >99% | 6 |
| | | GA-activated PEI | >99% | 11 |
| | | PEG-GA activated PEI | >99% | 12 |
| KRED-Ac-V2 | PEG 6000 | GA | >99% | 8 |
| | | GA-activated PEI | >99% | 13 |
| | | PEG-GA activated PEI | >99% | 13 |
| KRED-Cm | Acetonitrile | GA | >97% | 3 |
| | | GA-activated PEI | >97% | 8 |
| | | PEG-GA activated PEI | >97% | 9 |
| | Ammonium sulfate | GA-activated PEI | >97% | 12 |
| | | PEG-GA activated PEI | >97% | 12 |
| | Acetonitrile | GA-activated PEI | >97% | 10 |
| | | PEG-GA activated PEI | >97% | 12 |
| | PEG 6000 | GA-activated PEI | >97% | 10 |
| | | PEG-GA activated PEI | >97% | 12 |

### Embodiment 7: aqueous phase reaction activity verification of ketoreductase-cofactor-PEI covalently immobilized enzyme aggregate

A ketoreductase-cofactor-PEI covalently immobilized enzyme is prepared by the same method as in Embodiment 2.

The enzymatic activity and stability detection of the immobilized enzyme are performed by the same method as in Embodiment 6. Test results are shown in the following table.

**Table 11: overview of aqueous phase reaction of ketoreductase-cofactor-PEI covalently immobilized enzyme**

| Enzyme | EI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| KRED-Ac | 1 | 0 | GA | 1 | >97% | 6 |
| | 3 | 3 | GA | 1 | >97% | 14 |
| | 3 | 5 | GA | 1 | >97% | 16 |
| | 3 | 10 | GA | 1 | >97% | 17 |
| | 3 | 10 | PEG-GA | 1 | >97% | 18 |
| KRED-Cm | 3 | 0 | GA | 1 | >97% | 6 |
| | 3 | 5 | GA | 1 | >97% | 19 |
| | 3 | 10 | GA | 1 | >97% | 23 |
| | 2 | 10 | PEG-GA | 0.2 | >97% | 24 |
| | 3 | 10 | PEG-GA | 0.5 | >97% | 25 |
| | 2 | 10 | PEG-GA | 1 | >97% | 25 |
| | 2 | 10 | PEG-GA | 2 | >97% | 24 |

### Embodiment 8: aqueous phase reaction activity verification of cyclohexanone monooxygenase-glutaraldehyde-activated PEI covalently immobilized enzyme aggregate

A cyclohexanone monooxygenase-glutaraldehyde-activated PEI covalently immobilized enzyme is prepared according to the method of Embodiment 1.

The activity of the immobilized enzyme is detected by reacting with the following substrate 5.

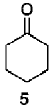

0.3 mL of IPA is loaded into a 10 ml reaction flask, and then 500 mg of the substrate 5 and 3 mL of 0.1 M PB (pH 8.0) containing 5 mg of a nicotinamide adenine dinucleoside phosphate (NADP+) are added, then 50 mg of alcohol dehydrogenase ADH-Tb free enzyme and 0.1 g of CHMO immobilized enzyme (wet, with a water content of 70-80%) are added. It is reacted at 30°C for 16-20 hours, and the conversion rate is tested. After each round of the reaction, the immobilized enzyme is separated, and reused in the next round of the reaction, and the recycling times are investigated. Results are shown in the following table.

**Table 12: cyclohexanone monooxygenase-glutaraldehyde-activated PEI covalently immobilized enzyme activity and recycling times**

| Enzyme | Precipitating agent | Cross-linking agent | Conversion rate (%) | Recycling times |
|---|---|---|---|---|
| CHMO-Bp | Ammonium sulfate | GA | >99% | 4 |
| | | GA-activated PEI | >99% | 7 |
| | | PEG-GA activated PEI | >99% | 9 |
| CHMO-Rr | PEG 6000 | GA | >99% | 6 |
| | | GA-activated PEI | >99% | 11 |
| | | PEG-GA activated PEI | >99% | 12 |
| CHMO-Rr-V1 | PEG 6000 | GA | >99% | 8 |
| | | GA-activated PEI | >99% | 15 |
| | | PEG-GA activated PEI | >99% | 15 |
| CHMO-Rr-V2 | PEG 6000 | GA | >99% | 8 |
| | | GA-activated PEI | >99% | 16 |
| | | PEG-GA activated PEI | >99% | 17 |
| CHMO-Rs | PEG 6000 | GA | >99% | 8 |
| | | GA-activated PEI | >99% | 13 |
| | | PEG-GA activated PEI | >99% | 13 |
| CHMO-Rs-V1 | PEG 6000 | GA | >99% | 12 |
| | | GA-activated PEI | >99% | 16 |
| | | PEG-GA activated PEI | >99% | 16 |
| CHMO-Rs-V2 | PEG 6000 | GA | >99% | 12 |
| | | GA-activated PEI | >99% | 17 |
| | | PEG-GA activated PEI | >99% | 17 |

### Embodiment 9: aqueous phase reaction activity verification of cyclohexanone monooxygenase-cofactor-activated PEI covalently immobilized enzyme aggregate

Same as in Embodiment 2, a cyclohexanone monooxygenase-cofactor activated PEI covalently immobilized enzyme aggregate is prepared.

Same as in Embodiment 8, the immobilized enzyme activity and the recycling times are detected, and results are shown in the following table.

**Table 13: cyclohexanone monooxygenase-cofactor-activated PEI covalently immobilized enzyme activity and recycling times**

| Enzyme | EI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| CHMO-Bp | 3 | 10 | GA | 1 | >97% | 9 |
| | 3 | 10 | GA | 1 | >97% | 10 |
| | 3 | 10 | PEG-GA | 1 | >97% | 13 |
| CHMO-Rr | 3 | 10 | GA | 1 | >97% | 11 |
| | 3 | 10 | PEG-GA | 1 | >97% | 11 |
| CHMO-Rr-V1 | 3 | 10 | GA | 1 | >97% | 18 |
| CHMO-Rr-V2 | 3 | 10 | GA | 1 | >97% | 20 |
| CHMO-Rs | 3 | 10 | GA | 1 | >97% | 9 |
| | 3 | 10 | PEG-GA | 0.2 | >97% | 7 |
| | 3 | 10 | PEG-GA | 0.5 | >97% | 10 |
| | 3 | 10 | PEG-GA | 1 | >97% | 12 |
| | 3 | 10 | PEG-GA | 2 | >97% | 6 |
| CHMO-Rs-V1 | 3 | 10 | GA | 1 | >97% | 16 |
| CHMO-Rs-V2 | 3 | 10 | GA | 1 | >97% | 18 |

### Embodiment 10: aqueous phase reaction activity verification of ene-reductase-glutaraldehyde-activated PEI covalently immobilized enzyme aggregate

An ene-reductase-glutaraldehyde-activated PEI covalently immobilized enzyme is prepared according to the method of Embodiment 1.

The activity of the immobilized enzyme and the recycling times are detected by performing a reaction with the following substrate 6.

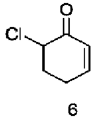

3 mL of 0.1 M PB (pH 7.0-8.0) is loaded into a 10 ml reaction flask, then 100 mg of the substrate 6 is added, and 10 mg of NAD(P)+, 80 mg of an ammonium formate, 20 mg of a formate dehydrogenase (FDH) and 100 mg of an ene-reductase (ERED) (wet, with a water content of 70-80%) are added. It is reacted at 30°C for 16-20 hours, and the conversion rate is tested. After each round of the reaction, the immobilized enzyme is separated, and reused in the next round of the reaction, and the recycling times are investigated. Test results are shown in the following table.

**Table 14: ene-reductase-glutaraldehyde-activated PEI covalently immobilized enzyme activity and recycling times**

| Enzyme | Precipitating agent | Cross-linking agent | Conversion rate (%) | Recycling times |
|---|---|---|---|---|
| ERED-Sc | Ammonium sulfate | GA | >99% | 8 |
| | | GA-activated PEI | >99% | 14 |
| | | PEG-GA activated PEI | >99% | 15 |
| ERED-Chr | PEG 6000 | GA | >99% | 12 |
| | | GA-activated PEI | >99% | 19 |
| | | PEG-GA activated PEI | >99% | 22 |

### Embodiment 11: aqueous phase reaction activity verification of ene-reductase-cofactor-PEI covalently immobilized enzyme aggregate

An ene-reductase-cofactor-PEI covalently immobilized enzyme is prepared by the same method as in Embodiment 2.

The enzymatic activity and stability detection of the immobilized enzyme are performed by the same method as in Embodiment 10, and results are shown in the following table.

**Table 15: ene-reductase-cofactor-PEI covalently immobilized enzyme activity and recycling times**

| Enzyme | PEI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| ERED-Sc | 3 | 10 | GA | 1 | >97% | 9 |
| | 3 | 10 | GA | 1 | >97% | 12 |
| | 3 | 10 | PEG-GA | 1 | >97% | 13 |
| ERED-Chr | 3 | 10 | GA | 1 | >97% | 8 |
| | 2 | 10 | PEG-GA | 1 | >97% | 14 |
| | 3 | 10 | PEG-GA | 1 | >97% | 15 |

### Embodiment 12: aqueous phase reaction activity verification of nitrilase-glutaraldehyde-activated PEI covalently immobilized enzyme aggregate

A nitrilase-glutaraldehyde-activated PEI covalently immobilized enzyme is prepared according to the method of Embodiment 1.

The activity and recycling times of the immobilized enzyme are tested by performing a reaction with the following substrate 7.

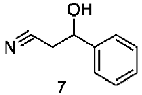

2 mL of 0.1 M PB buffer (pH 7.0-8.0) is added to a 10 mL reaction flask, and 100 mg of the above substrate 9 is added, and then 200 mg of an NIT immobilized enzyme (wet, with a water content of 70-80%) is added. After 16 hours of the reaction at 30°C, the conversion rate is detected. After each round of the reaction, the immobilized enzyme is separated, and reused in the next round of the reaction, and the recycling times are investigated. Test results are shown in the following table.

**Table 16: Nitrilase-glutaraldehyde-activated PEI covalently immobilized enzyme activity and recycling times**

| Enzyme | Precipitating agent | Cross-linking agent | Conversion rate (%) | Recycling times |
|---|---|---|---|---|
| NIT-An | Ammonium sulfate | GA | >99% | 4 |
| | | GA-activated PEI | >99% | 9 |
| | | PEG-GA activated PEI | >99% | 10 |
| NIT-Nc | PEG 6000 | GA | >99% | 4 |
| | | GA-activated PEI | >99% | 11 |
| | | PEG-GA activated PEI | >99% | 13 |

### Embodiment 13: aqueous phase reaction activity verification of nitrilase-cofactor-PEI covalently immobilized enzyme aggregate

A nitrilase-cofactor-PEI covalently immobilized enzyme is prepared by the same method as in Embodiment 2.

The enzyme activity and stability detection of the immobilized enzyme are performed by the same method as in Embodiment 12, and results are shown in the following table.

**Table 17: nitrilase-cofactor-PEI covalently immobilized enzyme activity and recycling times**

| Enzyme | PEI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| NIT-An | 1 | 10 | GA | 1 | >97% | 8 |
| | 3 | 10 | GA | 1 | >97% | 11 |
| | 3 | 10 | PEG-GA | 1 | >97% | 12 |
| NIT-Nc | 3 | 10 | GA | 1 | >97% | 10 |
| | 3 | 10 | PEG-GA | 0.2 | >97% | 12 |
| | 3 | 10 | PEG-GA | 0.5 | >97% | 16 |
| | 3 | 10 | PEG-GA | 1 | >97% | 16 |
| | 3 | 10 | PEG-GA | 2 | >97% | 11 |

### Embodiment 14: aqueous phase reaction activity verification of imine reductase-glutaraldehyde-activated PEI covalently immobilized enzyme aggregate

An imine reductase-glutaraldehyde-activated PEI covalently immobilized enzyme is prepared according to the method of Embodiment 1.

The activity of the immobilized enzyme is detected by reacting with the following substrate 8.

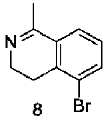

2 mL of 0.1 M PB buffer (pH 7.0-8.0) is added to a 10 mL reaction ball, and then 100 mg of the above substrate 8, 10 mg of NAD+, 60 mg of an ammonium formate, 10 mg of FDH, and 100 mg of IRED immobilized enzyme (wet, with a water content of 70-80%) are added. After 20 hours of the reaction at 30°C, the conversion rate is detected. After each round of the reaction, the immobilized enzyme is separated, and reused in the next round of the reaction, and the recycling times are investigated. Test results are shown in the following table.

**Table 18: imine reductase-glutaraldehyde-activated PEI covalently immobilized enzyme activity and recycling times**

| Enzyme | Precipitating agent | Cross-linking agent | Conversion rate (%) | Recycling times |
|---|---|---|---|---|
| IRED-Str | Ammonium sulfate | GA | >99% | 10 |
| | | GA-activated PEI | >99% | 17 |
| | | PEG-GA activated PEI | >99% | 17 |
| IRED-Bc | PEG 6000 | GA | >99% | 12 |
| | | GA-activated PEI | >99% | 20 |
| | | PEG-GA activated PEI | >99% | 20 |

### Embodiment 15: aqueous phase reaction activity verification of imine reductase-cofactor-PEI covalently immobilized enzyme aggregate

An imine reductase-cofactor-PEI covalently immobilized enzyme is prepared by the same method as in Embodiment 2.

The enzyme activity and stability detection of the immobilized enzyme are performed by the same method as in Embodiment 14, and results are shown in the following table.

**Table 19: imine reductase cofactor-PEI covalently immobilized enzyme activity and recycling times**

| Enzyme | PEI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| IRED-Str | 1 | 10 | GA | 1 | >97% | 12 |
| | 3 | 10 | GA | 1 | >97% | 15 |
| | 3 | 10 | PEG-GA | 1 | >97% | 17 |
| IRED-Bc | 3 | 10 | GA | 1 | >97% | 16 |
| | 3 | 10 | PEG-GA | 1 | >97% | 17 |

### Embodiment 16: aqueous phase reaction activity verification of ammonia lyase-glutaraldehyde-activated PEI covalently immobilized enzyme aggregate

An ammonia lyase-glutaraldehyde-activated PEI covalently immobilized enzyme is prepared according to the method of Embodiment 1.

The activity and recycling times of the immobilized enzyme are detected by performing a reaction with the following substrate 9.

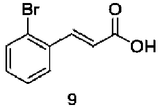

8 mL of 4 M ammonium carbamate aqueous solution (pH 9.0-9.5) is added into a 10 mL reaction flask, and 100 mg of the above substrate 9 is added, and then 200 mg of the ammonia lyase immobilized enzyme (wet, with a water content of 70-80%) is added. After 16-20 hours of the reaction at 30°C, the conversion rate is detected. After each round of the reaction, the immobilized enzyme is separated, and reused in the next round of the reaction, and the recycling times are investigated. Test results are shown in the following table.

**Table 20: ammonia lyase-glutaraldehyde-activated PEI covalently immobilized enzyme activity and recycling times**

| Enzyme | Enzyme | Cross-linking agent | Conversion rate (%) | Recycling times |
|---|---|---|---|---|
| PAL-An | Ammonium sulfate | GA | >99% | 10 |
| | | Ammonium sulfate | >99% | 17 |
| | | PEG-GA activated PEI | >99% | 17 |
| PAL-Ss | PEG 6000 | GA | >99% | 12 |
| | | Ammonium sulfate | >99% | 20 |
| | | PEG-GA activated PEI | >99% | 20 |

### Embodiment 17: aqueous phase reaction activity verification of ammonia lyase-cofactor-PEI covalently immobilized enzyme aggregate

An ammonia lyase-cofactor-PEI covalently immobilized enzyme is prepared by the same method as in Embodiment 2.

The enzyme activity and stability detection of the immobilized enzyme are performed by the same method as in Embodiment 16, and results are shown in the following table.

**Table 21: ammonia lyase-cofactor-PEI covalently immobilized enzyme activity and recycling times**

| Enzyme | PEI concentration, g/100mL | Cofactor NAD+ concentration, mg/mL | Cross-linking agent | Cross-linking agent concentration, g/100 mL | Conversion rate (%) | Recycling times (times) |
|---|---|---|---|---|---|---|
| PAL-An | 1 | 10 | GA | 1 | >97% | 12 |
| | 3 | 10 | GA | 1 | >97% | 15 |
| | 3 | 10 | PEG-GA | 1 | >97% | 17 |
| PAL-Ss | 3 | 10 | GA | 1 | >97% | 16 |
| | 3 | 10 | PEG-GA | 1 | >97% | 17 |

### Embodiment 18: aqueous phase reaction activity verification of amino acid dehydrogenase-glutaraldehyde-activated PEI covalently immobilized enzyme aggregate

An amino acid dehydrogenase-glutaraldehyde-activated PEI covalently immobilized enzyme is prepared according to the method of Embodiment 1.

The activity of the immobilized enzyme and the recycling times are detected by performing a reaction with the following substrates 10, 11 or 12.

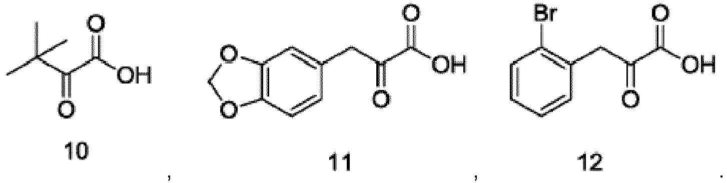

In a 10 mL reaction flask, 5 mL of 0.1 M Tris-Cl buffer (pH 8.0-9.0) is added, 100 mg of the main material 10, 11 or 12 is dissolved, and 108 mg of an ammonium chloride is added to adjust pH to 7.5-8.0, then 10-50 mg of NAD+, 50 mg of GDH and 100 mg of an immobilized AADH wet enzyme are added, and stirred at 30°C for 16-20 hours. The conversion rate of a system is detected by HPLC, and reaction data is as follows.

**Table 22: aqueous reaction overview of amino acid dehydrogenase-glutaraldehyde-activated PEI covalently immobilized enzyme**

| Enzyme | Precipitating agent | Cross-linking agent | Conversion rate (%) | Recycling times |
|---|---|---|---|---|
| AADH-Bc | | GA | >99% | 5 |
| | Ammonium sulfate | GA-activated PEI | >99% | 18 |
| | | PEG-GA activated PEI | >99% | 20 |
| | Acetonitrile | GA-activated PEI | >99% | 9 |
| | | PEG-GA activated PEI | >99% | 11 |
| | PEG 6000 | GA-activated PEI | >99% | 12 |
| | | PEG-GA activated PEI | >99% | 16 |
| AADH-Bs | Ethyl alcohol | GA-activated PEI | >99% | 8 |
| | | PEG-GA activated PEI | >99% | 9 |
| | Ammonium sulfate | GA | >99% | 6 |
| | | PEG-GA activated PEI | >99% | >15 |
| | Acetonitrile | GA-activated PEI | >99% | 8 |
| | | PEG-GA activated PEI | >99% | 8 |
| | PEG 6000 | GA-activated PEI | >99% | 19 |
| | | PEG-GA activated PEI | >99% | 20 |

### Embodiment 19: aqueous phase reaction activity verification of amino acid dehydrogenase-cofactor-activated PEI covalently immobilized enzyme aggregate

An amino acid dehydrogenase-cofactor-PEI covalently immobilized enzyme is prepared by the same method as in Embodiment 2.

The enzyme activity and stability detection of the immobilized enzyme are performed by the same method as in Embodiment 18, and results are shown in the following table.

**Table 23: aqueous reaction overview of amino acid dehydrogenase-cofactor-activated PEI covalently immobilized enzyme**

| Enzyme | PEI concentration, g/100mL | Cofactor NAD+ concentration, mg/mL | Cross-linking agent | Cross-linking agent concentration, g/100mL | Conversion rate (%) | Recycling times (times) |
|---|---|---|---|---|---|---|
| AADH-Bc | 1 | 10 | GA | 1 | >97% | 8 |
| | 3 | 10 | GA | 1 | >97% | 11 |
| | 3 | 10 | PEG-GA | 1 | >97% | 13 |
| AADH-Bs | 3 | 10 | GA | 1 | >97% | 10 |
| | 3 | 10 | PEG-GA | 0.2 | >97% | 10 |
| | 3 | 10 | PEG-GA | 0.5 | >97% | 13 |
| | 3 | 10 | PEG-GA | 1 | >97% | 14 |
| | 3 | 10 | PEG-GA | 2 | >97% | 10 |

### Embodiment 20: Co-immobilization of three enzymes TA, LDH and FDH covalently immobilized with glutaraldehyde-activated PEI

Immobilization: 10 mL of enzymes (TA:LDH:FDH) are mixed in proportion, placed in a round-bottomed shake flask, cooled to 5-10°C, and mixed for 10 min. A precipitating agent such as an ammonium sulfate (45%-65%), or PEG (20%-60%) is slowly added, and mixed for 30-60 min, to obtain a precipitating enzyme. 15 mL of activated PEI solution is added dropwise to the precipitating enzyme, and mixed for 10-30 min, after standing for 1-2 hours, it is centrifuged. An insoluble precipitate is collected, and sieved by a 30-mesh sieve. After being air-dried in a natural environment, it is soaked with 0.1 M PB (pH7.5, containing 0.5 M NaCl) for 3 hours, and then washed with 0.1 M PB (pH7.5).

The obtained co-immobilized enzyme is tested for the enzyme activity as follows.

In a 10 mL reaction flask, 5 mL of 0.1 M PB (pH 8.0) is added, 100 mg of a main raw material 12 is dissolved, 80 mg of a methionine and 5 mg of PLP are added, pH is adjusted to 7.5-8.0, and then 5 mg of NAD+, and 100 mg of the co-immobilized enzyme (wet, with a water content of 70-80%) are added. It is stirred at 30°C for 16-20 hours. The conversion rate of a system is detected by HPLC, and reaction data is as follows.

**Table 24:**

| Enzyme | Precipitating agent | Cross-linking agent | Conversion rate (%) | Conversion rate (%) |
|---|---|---|---|---|
| TA-Bt+LDH+FDH | Ammonium sulfate | GA | >99% | 7 |
| | | GA-activated PEI | >99% | 22 |
| | | PEG-GA activated PEI | >99% | 22 |
| | PEG 6000 | GA | >99% | 8 |
| | | GA-activated PEI | >99% | 22 |
| | | PEG-GA activated PEI | >99% | 23 |

### Embodiment 21: Application of transaminase-glutaraldehyde-modified PEI covalently immobilized enzyme in packed bed continuous reaction

In Embodiment 3, a transaminase TA-Cv-glutaraldehyde-modified PEI covalently immobilized enzyme is filled into a column reactor with a column volume of 120 mL, and the amount of the immobilized enzyme (wet, with a water content of 70-80%) is 90 g.

500 g of the substrate 1 is dissolved with 1.5 L of methanol, 4 eq of an isopropylamine hydrochloride (1.8 L of 6 M isopropylamine hydrochloride aqueous solution) and 5 g of PLP are added without adding a PB buffer (0.1 M, pH 8.0), the volume is fixed to 5 L.

The flow rate is set to 0.6 mL/min, namely the retention time is 200 min, and a continuous reaction is performed. The conversion rate of an effluent at an outlet end is detected.

### Embodiment 22: Application of transaminase-cofactor-modified PEI immobilized enzyme in continuous stirred tank reaction

60 g of a transaminase-cofactor-modified PEI immobilized enzyme prepared by the same method as in Embodiment 2 is added to a 200 mL reactor, and 150 mL of a phosphate buffer is added.

500 g of a substrate 1 is added with 3.2 L of PB (0.1 M, pH 7.0), 1.8 L of isopropylamine hydrochloride aqueous solution (6 M) and 5 g of PLP, and suspension is prepared by beating.

The substrate suspension is continuously added to the reaction flask at a rate of 0.4 mL/min (namely the retention time is 500 min), and a reaction system is extracted at an outlet at the same flow rate (a filter head is added at a tail end of a pipe, to prevent the immobilized enzyme from being extracted). Under this condition, the conversion rate may reach more than 90%, and the conversion rate is basically not reduced after continuous operation for 600 hours.

### Embodiment 23: Application of TA, LDH and FDH three enzymes and glutaraldehyde-activated PEI covalently immobilized enzyme in continuous stirred tank reaction

The same co-immobilized enzyme as in Embodiment 20 is used, 70 g of the co-immobilized enzyme of transaminase TA-Bt and coenzyme LDH and FDH is added to a 200 mL reactor, and 150 mL of a phosphate buffer is added.

500 g of a substrate 12, and 108 mg of an ammonium chloride are dissolve with 4.5 L of a PB buffer (0.1 M, pH 8.0), pH is adjusted to 7.5-8.0 with sodium hydroxide solution, then 10-50 mg of NAD+, and 80 mg of an ammonium formate are added, and finally the volume is fixed to 5 L with the PB buffer.

The substrate is continuously added to a continuous stirring tank at a rate of 0.8 mL/min (namely the retention time is 250 min), and a reaction system is extracted at an outlet at the same flow rate (a filter head is added at a tail end of a pipeline, to prevent the immobilized enzyme from being extracted). Under this condition, the conversion rate may reach more than 92%, and the conversion rate is basically not reduced after continuous operation for 600 hours.

### Embodiment 24

In the same method as in Embodiment 2, 8 g of the prepared ketoreductase KRED-Ac immobilized enzyme (wet, with a water content of 70-80%) is filled into a 10 mL column reactor.

100 g of a substrate 3 is dissolved with 0.3 L of isopropanol, and dissolved with 0.7 L of a PB buffer (0.1 M, pH 7.0), and then 0.1 g of NAD⁺ is added.

The flow rate is set to 0.05 mL/min, namely the retention time is 200 min, and a continuous reaction is performed, An effluent at an outlet is tested for the conversion rate, the conversion rate is >90%, and the conversion rate is basically not reduced after continuous operation for 500 hours.

From the above descriptions, it may be seen that the above embodiments of the present invention achieve the following technical effects: by immobilizing the enzyme with PEI, the enzyme is immobilized between the polymeric network-like structures formed by PEI, the mechanical stability is improved, the immobilization of the enzyme is achieved, and in the process of immobilization, the enzyme is avoided from directly covalently binding to the cross-linking agent such as the glutaraldehyde so as to reduce the activity.

The above are only preferred embodiments of the present invention, and are not intended to limit the present invention. For those skilled in the art, the present invention may have various modifications and changes. Any modifications, equivalent replacements, improvements and the like made within the spirit and principle of the present invention shall be included within a scope of protection of the present invention.

## Claims

1. An immobilized enzyme, wherein the immobilized enzyme comprises an activated cationic polymer and an enzyme covalent-bonded on the activated cationic polymer,
wherein the activated cationic polymer is a polyethylene imine (PEI) activated by a cross-linking agent or one or more cofactors, and the cross-linking agent is modified or not modified by a polyol;
the enzyme is selected from any one of the group consisting of: a transaminase, a ketoreductase, a monooxygenase, an ammonia lyase, an ene reductase, an imine reductase, an amino acid dehydrogenase and a nitrilase.

2. The immobilized enzyme as claimed in claim 1, wherein the transaminase is derived from *B.thuringiensis, Arthrobacter citreus* or *Chromobacterium violaceum DSM30191;*
the ketoreductase is derived from *Acetobacter sp. CCTCC M209061* or *Candida macedoniensis. AKU4588;*
the monooxygenase *is a cyclohexanone monooxygenase derived from Brachymonas petroleovorans or Rhodococcus ruber-SD1;*
the ammonia lyase is a phenylalanine ammonia lyase derived from *Photorhabdus luminescens* or *Solenostemon scutellarioides*;
the ene reductase is derived from *Saccharomyces cerevisiae* or *Chryseobacterium sp. CA49;*
the imine reductase is derived from *Streptomyces sp. or Bacillus cereus;*
the amino acid dehydrogenase is a leucine dehydrogenase derived from *Bacillus cereus* or a phenylalanine dehydrogenase derived from *Bacillus sphaericus;* and
the nitrilase is derived from *Aspergillus niger* CBS 513.88 or *Neurospora crassa* OR74A.

3. The immobilized enzyme as claimed in claim 1 or 2, wherein a molecular weight of the PEI is 3 KDa to 600 KDa.

4. The immobilized enzyme as claimed in claim 1 or 2, wherein a mass ratio of the enzyme and the PEI in the immobilized enzyme is 0.1-1.5:1.

5. The immobilized enzyme as claimed in claim 1 or 2, wherein the one or more cofactors are selected from a pyridoxal phosphate (PLP), a nicotinamide adenine dinucleotide (NAD) and a nicotinamide adenine dinucleotide phosphate (NADP), the cross-linking agent is glutaraldehyde.

6. The immobilized enzyme as claimed in claim 1 or 2, wherein the polyol is PEG.

7. The immobilized enzyme as claimed in claim 6, wherein the polyol is PEG 400-6000.

8. The immobilized enzyme as claimed in claim 5, wherein there is one or more enzymes, there is one or more cofactors, and the one or more cofactors correspond to the one or more enzymes.

9. The immobilized enzyme as claimed in claim 5, wherein a mass ratio of the one or more cofactors and the PEI in the immobilized enzyme is 1-80:120.

10. The immobilized enzyme as claimed in claim 5, wherein the mass ratio of the one or more cofactors and the PEI in the immobilized enzyme is 1-60:100.

11. A preparation method for an immobilized enzyme, wherein the preparation method comprises:
activating a cationic polymer with an activating agent, to obtain an activated cationic polymer;
enabling an enzyme to be immobilized with the activated cationic polymer, to obtain the immobilized enzyme;
wherein, the activating agent is a glutaraldehyde modified or not modified by a polyol, or one or more cofactors; and
the enzyme is selected from any one of the group consisting of: a transaminase, a ketoreductase, a monooxygenase, an ammonia lyase, an ene reductase, an imine reductase, an amino acid dehydrogenase and a nitrilase.

12. The preparation method as claimed in claim 11, wherein before activating the PEI with the activating agent, the preparation method further comprises a step of performing pre-treatment on the PEI.

13. The preparation method as claimed in claim 11, wherein the pre-treatment comprises:
diluting the PEI with pure water until a final concentration is 1 g/mL to 8 g/mL, to obtain a diluted PEI; and
adjusting a pH of the diluted PEI to be 5-11, to obtain the PEI after the pre-treatment.

14. The preparation method as claimed in claim 12, wherein the pre-treatment comprises:
diluting the PEI with pure water until a final concentration is 1 g/mL to 5 g/mL, to obtain a diluted PEI; and
adjusting a pH of the diluted PEI to be 5-11, to obtain the PEI after the pre-treatment.

15. The preparation method as claimed in claim 12, wherein the pre-treatment comprises:
diluting the PEI with pure water until a final concentration is 1 g/mL to 8 g/mL, to obtain a diluted PEI; and
adjusting a pH of the diluted PEI to be 7.0-10.0, to obtain the PEI after the pre-treatment.

16. The preparation method as claimed in claim 12, wherein the pre-treatment comprises:
diluting the PEI with pure water until a final concentration is 1 g/mL to 5 g/mL, to obtain a diluted PEI; and
adjusting a pH of the diluted PEI to be 7.0-10.0, to obtain the PEI after the pre-treatment.

17. The preparation method as claimed in any one of claims 13 to 16, wherein the enzyme is a precipitated enzyme, the cross-linking agent is glutaraldehyde, and the activating agent is the glutaraldehyde or the PEG-modified glutaraldehyde.

18. The preparation method as claimed in claim 17, wherein in the step of the pre-treatment, adjusting the pH of the diluted PEI to be 5.0-11.0, preferably 7.0-10.0, to obtain the PEI after the pre-treatment.

19. The preparation method as claimed in claim 17, wherein adjusting the pH of the diluted PEI to be 7.0-10.0.

20. The preparation method as claimed in claim 17, wherein the step of activating the PEI with the activating agent to obtain the activated PEI comprises:
dropwise adding the glutaraldehyde or the PEG-modified glutaraldehyde to the PEI after the pre-treatment, and controlling a final concentration of the glutaraldehyde or the PEG-modified glutaraldehyde in a system to be 0.1 g/mL to 4 g/mL, to obtain the activated PEI.

21. he preparation method as claimed in claim 17, wherein the step of activating the PEI with the activating agent to obtain the activated PEI comprises:
dropwise adding the glutaraldehyde or the PEG-modified glutaraldehyde to the PEI after the pre-treatment, and controlling a final concentration of the glutaraldehyde or the PEG-modified glutaraldehyde in a system to be 0.3 g/mL to 2 g/mL, to obtain the activated PEI.

22. The preparation method as claimed in claim 17, wherein the precipitated enzyme is obtained by precipitating the enzyme with a precipitant, and the precipitant is selected from an organic solvent, an ammonium sulfate, PEG 400-6000 or a PEI of which the molecular weight is 3 KDa to 70 KDa.

23. The preparation method as claimed in claim 20 or 21, wherein in a process of dropwise adding the glutaraldehyde or the PEG-modified glutaraldehyde to the PEI after the pre-treatment, the rate of dropping of the glutaraldehyde or the PEG-modified glutaraldehyde is controlled to be 10 to 50 mL/min.

24. The preparation method as claimed in claim 23, wherein in the process of dropwise adding, further comprises a stirring step, a temperature of the stirring is 4 to 25 DEG C, a speed of the stirring is 50 to 400 rpm, and time of the stirring is 1 to 12 h.

25. The preparation method as claimed in claim 24, wherein the speed of the stirring is 100 to 300 rpm.

26. The preparation method as claimed in claim 24, wherein the time of the stirring is 2 to 10 h.

27. The preparation method as claimed in claim 17, wherein enabling the enzyme to be immobilized with the activated PEI to obtain the immobilized enzyme comprises:
dropwise adding the activated PEI to the precipitated enzyme, to obtain a mixture;
successively performing incubation, centrifuging, sieving and drying treatment on the mixture, to obtain the immobilized enzyme.

28. The preparation method as claimed in claim 27, wherein a mass ratio of the enzyme and the activated PEI is 0.2-1:1.

29. The preparation method as claimed in claim 27, wherein time of the incubation is 1 to 5 hours.

30. The preparation method as claimed in claim 27, wherein the sieving is to pass through #20 to # 50 seive mesh.

31. The preparation method as claimed in claim 27, wherein the drying is by open air drying.

32. The preparation method as claimed in claim 27, wherein more preferably overnight drying.

33. The preparation method as claimed in claim 27, wherein after the mixture is dried, and before the PEI immobilized enzyme is obtained, the preparation method further comprises:
a step of washing the dried mixture with phosphate buffer, water and the phosphate buffer successively, wherein each type of washing is repeated for 3 to 5 times, and a pH of the phosphate buffer is 7.0 to 8.0.

34. The preparation method as claimed in any one of claims 13-16, wherein the enzyme is a free enzyme, the activating agent is one or more cofactors, and the one or more cofactors are selected from PLP, NAD and NADP.

35. The preparation method as claimed in claim 34, wherein in the step of the pre-treatment, with purified water, adjusting concentration of the diluted PEI to be 1 g/mL to 8 g/mL, wherein a pH is 6 to 11, to obtain the PEI after the pre-treatment.

36. The preparation method as claimed in claim 35, wherein adjusting concentration of the diluted PEI to be 1 g/mL to 3 g/mL.

37. The preparation method as claimed in claim 35, wherein the pH is 8.0 to 11.0.

38. The preparation method as claimed in claim 35, wherein the step of activating the PEI with the activating agent to obtain the activated PEI comprises:
adding the one or more cofactors to the PEI after the pre-treatment, and
controlling a final concentration of the cofactor to be 0.3 mg/mL to 20 mg/mL, and
then stirring for 10 to 600 min, to obtain the activated PEI.

39. The preparation method as claimed in claim 38, wherein controlling the final concentration of the one or more cofactors is 1 mg/mL to 10 mg/mL.

40. The preparation method as claimed in claim 38, wherein stirring for 30 to 180 min.

41. The preparation method as claimed in claim 34, wherein the free enzyme is an enzyme solution of one enzyme or a mixed solution of multiple enzymes, and the one or more cofactors are corresponding to one enzyme or multiple enzymes.

42. The preparation method as claimed in claim 38, wherein the step of enabling the enzyme to be immobilized with the activated PEI to obtain the immobilized enzyme comprises:
at 4 to 25 DEG C, dropwise adding the activated PEI to an enzyme solution of the free enzyme, to obtain a PEI adsorbed enzyme;
dropwise adding a cross-linking agent to the PEI adsorbed enzyme and performing immobilization, to obtain the PEI immobilized enzyme.

43. The preparation method as claimed in claim 42, wherein a concentration of the free enzyme is 0.05 to 0.3 g/mL.

44. The preparation method as claimed in claim 42, wherein a mass ratio of the PEI and the free enzyme is 0.2-1:1.

45. The preparation method as claimed in claim 42, wherein dropwise adding the cross-linking agent to the PEI adsorbed enzyme and performing the immobilization to obtain the PEI immobilized enzyme comprises:
dropwise adding the cross-linking agent to the PEI adsorbed enzyme, and controlling a final concentration of the cross-linking agent to be 0.2 g/m to 1 g/mL, to obtain a substance to be immobilized;
successively performing incubation, centrifuging and drying on the substance to be immobilized, to obtain a dried enzyme;
washing the dried enzyme, to obtain the immobilized enzyme.

46. The preparation method as claimed in claim 45, wherein the time of the incubattion is 10 to 300 min, the drying is open air drying, the washing is washing for 3 to 5 times with phosphate buffer, washing for 3 to 5 times with water and washing for 3 to 5 times with the phosphate buffer successively, and the phosphate buffer contains NaCl, wherein concentration of the NaCl is 0.5 to 1 M.

47. The preparation method as claimed in claim 45, wherein the time of the incubattion is 30 to 120 min.

48. The preparation method as claimed in claim 11, wherein the transaminase is derived from *B.thuringiensis, Arthrobacter citreus or Chromobacterium violaceum DSM30191;*
the ketoreductase is derived from *Acetobacter sp. CCTCC M209061 or Candida macedoniensis. AKU4588;*
the monooxygenase is a cyclohexanone monooxygenase derived from *Brachymonas petroleovorans or Rhodococcus ruber-SD1;*
the ammonia lyase is a phenylalanine ammonia lyase derived from *Photorhabdus luminescens or Solenostemon scutellarioides;*
the ene reductase is derived from *Saccharomyces cerevisiae or Chryseobacterium sp. CA49;*
the imine reductase is derived from *Streptomyces sp. or Bacillus cereus;*
the amino acid dehydrogenase is a leucine dehydrogenase derived from *Bacillus cereus* or a phenylalanine dehydrogenase derived from *Bacillus sphaericus;* and
the nitrilase is derived from *Aspergillus niger CBS 513.88 or Neurospora crassa OR74A.*

49. Use of the immobilized enzyme as claimed in any one of claims 1 to 10 or the immobilized enzyme prepared by the preparation method as claimed in any one of claims 11 to 48 in a bio-catalytic reaction.

50. The use as claimed in claim 49, wherein the bio-catalytic reaction is a batch reaction or a continuous reaction.
